# EUROPEAN PATENT SPECIFICATION

(11) **EP 4 177 355 B1**
(45) Date of publication and mention of the grant of the patent: **01.07.2026**
(21) Application number: 21834599.9
(22) Date of filing: 07.05.2021
(51) Int. Cl.: C12Q 1/6883

(54) **METHOD FOR DIAGNOSING DOWN'S SYNDROME BY USING DOWN'S SYNDROME-SPECIFIC EPIGENETIC MARKER**
VERFAHREN ZUR DIAGNOSE DES DOWN-SYNDROMS DURCH VERWENDUNG EINES DOWN-SYNDROM-SPEZIFISCHEN EPIGENETISCHEN MARKERS
PROCÉDÉ POUR DIAGNOSTIQUER LE SYNDROME DE DOWN EN UTILISANT UN MARQUEUR ÉPIGÉNÉTIQUE SPÉCIFIQUE AU SYNDROME DE DOWN

(30) Priority: 02.07.2020 KR 20200081685
(43) Date of publication of application: 10.05.2023
(73) Proprietor: Sungkwang Medical Foundation, Seoul 06135 (KR)
(72) Inventor: RYU, Hyun Mee, Seongnam-si, Gyeonggi-do 13497 (KR); LIM, Ji Hyae, Seongnam-si, Gyeonggi-do 13488 (KR)
(74) Representative: Weickmann & Weickmann PartmbB
(86) International application number: PCT/KR2021/005717
(87) International publication number: WO 2022/005010

(56) References cited:
- EP-A1- 3 330 382
- LIM JI HYAE: "Epigenome-wide base-resolution profiling of DNA methylation in chorionic villi of down syndrome fetuses by methyl-capture sequencing", 1 January 2019 (2019-01-01), XP009533500, Retrieved from the Internet <URL:http://www.riss.kr/link?id=T15033821>
- SHENGNAN JIN ET AL: "Global DNA Hypermethylation in Down Syndrome Placenta", PLOS GENETICS, vol. 9, no. 6, 1 January 2013 (2013-01-01), USA, pages e1003515 - 1, XP055248023, ISSN: 1553-7390, DOI: 10.1371/journal.pgen.1003515
- KRISTI KERKEL ET AL: "Altered DNA Methylation in Leukocytes with Trisomy 21", PLOS GENETICS, vol. 6, no. 11, 18 November 2010 (2010-11-18), pages 1 - 13, XP055139128, DOI: 10.1371/journal.pgen.1001212
- LIM JI HYAE ET AL: "Epigenome-wide base-resolution profiling of DNA methylation in chorionic villi of fetuses with Down syndrome by methyl-capture sequencing", CLINICAL EPIGENETICS, vol. 11, no. 1, 1 December 2019 (2019-12-01), London, UK, pages 1 - 11, XP055885529, ISSN: 1868-7075, Retrieved from the Internet <URL:http://link.springer.com/article/10.1186/s13148-019-0756-4/fulltext.html> DOI: 10.1186/s13148-019-0756-4
- OLD ROBERT W ET AL: "Candidate epigenetic biomarkers for non-invasive prenatal diagnosis of Down syndrome", REPRODUCTIVE BIOMEDICINE ONLINE, ELSEVIER, AMSTERDAM, NL, vol. 15, no. 2, 1 January 2007 (2007-01-01), pages 227 - 235, XP008144463, ISSN: 1472-6483, [retrieved on 20100518], DOI: 10.1016/S1472-6483(10)60713-4
- LIM JI HYAE: "Epigenome-wide base-resolution profiling of DNA methylation in chorionic villi of down syndrome fetuses by methyl-capture sequencing", PH.D. THESIS, vol. 11, 180, 1 January 2019 (2019-01-01), Korea, pages 1 - 60, XP009533500
- SHENGNAN JIN, YEW KOK LEE, YEN CHING LIM , ZEJUN ZHENG , XUEQIN MICHELLE LIN , DESMOND P. Y. NG , JOANNA D. HOLBROOK , HAI YANG LA: "Global DNA hypermethylation in down syndrome placenta", PLOS GENETICS, vol. 9, no. 6, e1003515, 6 June 2013 (2013-06-06), pages 1 - 9, XP055248023
- KRISTI KERKEL, NICOLE SCHUPF, KOTA HATTA, DEBORAH PANG, MARTHA SALAS, ALEXANDER KRATZ, MARK MINDEN, VUNDAVALLI MURTY, WARREN B. ZI: "Altered DNA Methylation in Leukocytes with Trisomy 21", PLOS GENETICS, vol. 6, no. 11, e1001212, 18 November 2010 (2010-11-18), pages 1 - 13, XP055139128, DOI: 10.1371/journal.pgen.1001212
- LIM JI HYAE, KANG YU-JUNG, LEE BOM YI, HAN YOU JUNG, CHUNG JIN HOON, KIM MOON YOUNG, KIM MIN HYOUNG, KIM JIN WOO, CHO YOUL-HEE, RY: "Epigenome-wide base-resolution profiling of DNA methylation in chorionic villi of fetuses with Down syndrome by methyl-capture sequencing", CLINICAL EPIGENETICS, vol. 11, no. 1, 1 December 2019 (2019-12-01), London, UK, pages 1 - 11, XP055885529, ISSN: 1868-7075, DOI: 10.1186/s13148-019-0756-4
- OLD ROBERT W; CREA FRANCESCO; PUSZYK WILLIAM; HULTÉN MAJ ANITA: "Candidate epigenetic biomarkers for non-invasive prenatal diagnosis of Down syndrome.", REPRODUCTIVE BIOMEDICINE ONLINE, vol. 15, no. 2, 1 January 2007 (2007-01-01), AMSTERDAM, NL , pages 227 - 235, XP008144463, ISSN: 1472-6483, DOI: 10.1016/S1472-6483(10)60713-4

## Description

### Technical Field

The present disclosure relates to a Down syndrome-specific epigenetic marker and a method of providing information for diagnosing Down syndrome and a method of diagnosing Down syndrome.

### Background Art

Mutations resulting from structural abnormalities in chromosomes result in abnormal development and death of embryos. However, in the case of Down syndrome (DS), a type of disease caused by the most common chromosomal abnormality, the embryo survives. According to the World Health Organization (WHO), Down syndrome, the most common genetic cause of developmental disabilities, has an estimated prevalence of 1 to 100 in 1,100 worldwide. Down syndrome exhibits more than 80 clinical features, including mental retardation, facial features, poor muscle development, and short stature, and is related to an increased risk of congenital heart disease, diabetes, leukemia, and other diseases.

A technique in the art for diagnosing Down syndrome is a method of analyzing the quantitative difference of cfDNA, a non-cellular DNA fragment present in plasma of pregnant women (next generation sequencing (NGS)-based non-invasive prenatal testing (NIPT)). This method shows high detection accuracy for Down syndrome and is quickly being applied to clinical practice, but expensive equipment and test consumables are required, and the analysis method is also complicated, making it difficult to apply in general laboratories, and there is a limitation in that the test is difficult to apply to all pregnant women due to the high costs. In addition, there is a method using single-nucleotide polymorphisms (SNPs) (PCR based NIPT), but there is no clear basis for the test accuracy, and a general use is limited because a special algorithm presented by each institution needs to be used. That is, for the above-mentioned chromosomal disease, research is actively being conducted on an analysis method capable of finding out the presence of the disease in advance through genetic analysis (Korean Patent Publication No. 10-2019-0003987), but is still incomplete. LIM JI HYAE ET AL (2019-02-01 and 2019-12-01) performed an epigenome-wide base-resolution profiling of DNA methylation in chorionic villi of fetuses with Down syndrome by methyl-capture sequencing. SHENGNAN JIN ET AL (2013-01-01) analyses the global DNA Hypermethylation in Down Syndrome Placenta. KRISTI KERKEL ET AL (2010-11-18) studied the altered DNA methylation in leukocytes with Trisomy 21. OLD R. AL (2007-01-01) provided epigenetic biomarkers for non-invasive prenatal diagnosis of Down syndrome.

With this background, the present inventors have completed the present disclosure, which may effectively test for Down syndrome through simple measurement of a methylation level of the genome, without the need to utilize existing expensive test equipment or special algorithms.

### Disclosure

### Technical Problem

The present disclosure provides a method of providing information for diagnosing Down syndrome, the method including: measuring a methylation level of a Down syndrome biomarker in a biological sample separated from a subject, wherein the biomarker is a first biomarker present on chromosome 21, a second biomarker present on a chromosome other than chromosome 21, or a combination thereof; comparing the measured methylation level of the biomarker with a methylation level of the first biomarker and the second biomarker in the biological sample separated from a normal control group; and determining a presence or a risk of Down syndrome by comparing the methylation levels.

### Technical Solution

The invention is defined in the appended claims. Disclosed, but not part of the invention, is a method of providing information for diagnosing Down syndrome, the method including: measuring a methylation level of a Down syndrome biomarker in a biological sample separated from a subject, wherein the biomarker is a first biomarker present on chromosome 21, a second biomarker present on a chromosome other than chromosome 21, or a combination thereof; comparing the measured methylation level of the biomarker with a methylation level of the first biomarker

The term "Down syndrome", used herein, refers to a kind of congenital genetic disease, in which one more full or partial copy of chromosome 21 is present than normal. Down syndrome causes delayed physical development and may be accompanied by facial deformities and intellectual disabilities. Most of the chromosomes of a patient with a Down syndrome, which are inherited from parents, are normal, but there may be one more copy of chromosome 21 due to nondisjunction of reproductive cells during pregnancy. The incidence rate is 0.1 % in 20-year-old mothers, but increases rapidly to 4 % in 45-year-old mothers, and the incidence rate shows a tendency to increase with the mother's age. Down syndrome may be confirmed by a prenatal diagnosis of the fetus during pregnancy, and may be determined through direct genetic testing after childbirth.

The term "diagnosis", used herein, refers to confirming a presence or characteristics of a pathological state, and may include determining whether or not Down syndrome has occurred or is likely to occur.

The term "methylation", used herein, may mean that a methyl group is added to the 5th carbon of a cytosine residue of DNA, and may mean that a methyl group is attached to a base constituting DNA. Preferably, an occurrence of methylation means an occurrence of methylation at the fifth carbon of a cytosine residue of a specific CpG site of a specific gene. When methylation occurs, binding of transcription factors is hindered, and therefore, expression of specific genes is inhibited, and conversely, when unmethylation or hypo-methylation occurs, expression of specific genes increases. In genomic DNA of mammalian cells, there is a fifth base called 5-methylcytosine (5-mC) with a methyl group attached to the fifth carbon of a cytosine ring, in addition to A, C, G, and T. Methylation of 5-methylcytosine occurs only at C of CG dinucleotide (5'-mCG-3') called CpG, and methylation of CpG inhibits expression of alu or transposon and genomic repetitive sequences. In addition, since 5-mC of CpG is easy to be naturally deaminated to become thymine (T), CpG is a site where most epigenetic changes occur frequently in mammalian cells.

The term "measurement of a methylation level", used herein, may include measuring a level of methylation of Down syndrome-related gene biomarkers in a biological sample in order to diagnose Down syndrome. The measurement of a methylation level is to measure methylation levels of CpG sites, and any method known in the art for measuring a methylation level may be used without limitation, but methylation-specific PCR, for example, methylation-specific polymerase chain reaction (MSP), real-time methylation-specific polymerase chain reaction, PCR using binding proteins specific for methylated DNA, quantitative PCR, PCR using methylation-specific specific peptide nucleic acid (PNA), or melting curve analysis may be used. Alternatively, a methylation level may be measured by methods such as DNA chip, pyrosequencing, bisulfite sequencing, and automatic sequencing such as methyl-capture sequencing (MC-Seq), etc., but is not limited thereto.

The term "differentially methylated CpG site (DMC)", used herein, may refer to a CpG site that exhibits different DNA methylation states depending on the stage of development, type of tissue, and a presence or absence of a disease. A region in a genome where DMCs repeatedly exist may be referred to as "differentially methylated CpG region (DMR)". Most DNA methylation occurs at the CpG site, in which C represents cytosine, G represents guanine, and p may represent a phosphodiester bond between the cytosine and the guanine. In normal human somatic cells, the CpG island in the housekeeping gene promoter region is unmethylated, and genes that are not expressed during development, such as imprinted genes and inactive genes on the X chromosome, are methylated.

The agent for measuring a methylation level may be an agent for confirming the presence or absence of methylation of a gene, and may be for measuring an amount of methylated genes. The agent for measuring a methylation level may include, for example, a compound or methylation-specific restriction enzyme (MSRE) that modifies unmethylated cytosine bases, a primer specific for a methylated sequence of the gene, and a primer specific for an unmethylated sequence. The compound that modifies the unmethylated cytosine base may be bisulfite, but is not limited thereto, and may be preferably sodium bisulfite. This method of detecting methylation of a promoter by modifying an unmethylated cytosine residue by using bisulfite is well known in the art. The methylation-specific restriction enzyme refers to an enzyme that selectively cuts nucleic acids according to the methylation state of its restriction site. For restriction enzymes that specifically cleave when the restriction site is unmethylated or hemimethylated, cleavage will not occur or will occur with significantly reduced efficiency when the restriction site is methylated. For restriction enzymes that specifically cleave when the restriction site is methylated, cleavage will not occur or will occur with significantly reduced efficiency when the restriction site is unmethylated.

Also disclosed but not part of the invention is a use of the first biomarker for diagnosing Down syndrome.

The first biomarker may be at least one selected from the group presented in Table 1 below.

**[Table 1]**

| **No.** | **Gene** | **Region in chromosome** |
|---|---|---|
| 1 | CHODL | chr21-19617176-19617247 |
| 2 | | chr21-19617336-19617406 |
| 3 | | chr21-19617673-19617752 |
| 4 | NCAM2 | chr21-22370043-22370078 |
| 5 | CYYR1 | chr21-27944498-27944673 |
| 6 | GRIK1 | chr21-31311263-31311314 |
| 7 | | chr21-31311387-31311511 |
| 8 | OLIG2 | chr21-34391993-34392227 |
| 9 | | chr21-34392448-34392476 |
| 10 | | chr21-34392696-34392868 |
| 11 | | chr21-34395135-34395176 |
| 12 | | chr21-34395490-34395566 |
| 13 | | chr21-34395876-34395965 |
| 14 | | chr21-34396297-34396346 |
| 15 | | chr21-34396460-34396728 |
| 16 | | chr21-34397003-34397119 |
| 17 | | chr21-34399292-34399362 |
| 18 | | chr21-34399427-34399529 |
| 19 | | chr21-34399681-34399714 |
| 20 | | chr21-34399848-34399903 |
| 21 | | chr21-34400145-34400212 |
| 22 | | chr21-34400714-34400988 |
| 23 | | chr21-34401366-34401379 |
| 24 | | chr21-34401534-34401769 |
| 25 | CLIC6 | chr21-36042254-36042285 |
| 26 | | chr21-36042533-36042735 |
| 27 | SIM2 | chr21-38067964-38068044 |
| 28 | | chr21-38068418-38068806 |
| 29 | | chr21-38069018-38069190 |
| 30 | | chr21-38069475-38070680 |
| 31 | | chr21-38072494-38072506 |
| 32 | | chr21-38073032-38073075 |
| 33 | | chr21-38073463-38073528 |
| 34 | | chr21-38073992-38074050 |
| 35 | | chr21-38074152-38074184 |
| 36 | | chr21-38074992-38075172 |
| 37 | | chr21-38076769-38077111 |
| 38 | | chr21-38077206-38077398 |
| 39 | | chr21-38078262-38078458 |
| 40 | | chr21-38079128-38079359 |
| 41 | | chr21-38079924-38079962 |
| 42 | | chr21-38079988-38080178 |
| 43 | | chr21-38081200-38081254 |
| 44 | | chr21-38081394-38081455 |
| 45 | | chr21-38083112-38083128 |
| 46 | HLCS | chr21-38352985-38353060 |
| 47 | | chr21-38353193-38353257 |
| 48 | MX2 | chr21-42741677-42741853 |
| 49 | MX1 | chr21-42798695-42798848 |
| 50 | TMPRSS2 | chr21-42878480-42878569 |
| 51 | SLC37A1, PDE9A | chr21-44011221-44011266 |
| 52 | CBS | chr21-44473401-44473497 |
| 53 | CRYAA | chr21-44588388-44588457 |
| 54 | C21orf2, TRPM2 | chr21-45769923-45770010 |
| 55 | TSPEAR | chr21-46126022-46126188 |
| 56 | | chr21-46126891-46127100 |
| 57 | | chr21-46128800-46128978 |
| 58 | | chr21-46129159-46129689 |
| 59 | LINC00162, SSR4P1 | chr21-46439211-46439237 |
| 60 | SLC19A1, LOC100129027 | chr21-47062136-47062158 |
| 61 | MCM3AP | chr21-47704178-47704210 |
| 62 | YBEY | chr21-47717843-47717867 |
| 63 | | chr21-47717931-47718023 |
| 64 | PRMT2, NONE | chr21-48087183-48087258 |
| 65 | ITSN1 | chr21-35246628-35246690 |

The term "CHODL" gene, used herein, refers to a gene encoding a chondrolectin protein. The exact function of the protein encoded by the gene is unknown, but the gene has been shown to be a marker of fast motor neurons in mice.

The term "NCAM2" gene, used herein, refers to a gene encoding a neural cell adhesion molecule 2 protein. The gene is known to be associated with a prion disease.

The term "CYYR1" gene, used herein, refers to a gene encoding cysteine and tyrosine-rich protein 1 (CYYR1). The function of the protein encoded by the gene has not been specifically known.

The term "GRIK1" gene, used herein, refers to a gene encoding a glutamate receptor, ionotropic, kainate 1 (GRIK1) protein. The gene encodes one of many subunits of an ionic glutamate receptor (GluR) that function as ligand-gated ion channels.

The term "OLIG2" gene, used herein, refers to a gene encoding an oligodendrocyte transcription factor protein. It is known that the expression of the gene is mainly restricted in the central nervous system, where the gene acts as both an anti-neurigenic and a neurigenic factor at different stages of development, and that the gene is mainly associated with brain tumors.

The term "CLIC6" gene, used herein, refers to a gene encoding chloride intracellular channel protein 6. The gene is known to interact with the dopamine receptor D3.

The term "SIM2" gene, used herein, refers to a gene encoding a single-minded homolog 2 protein. The protein encoded by the gene is known to play an important role in the development of the midline of the central nervous system as well as the construction of the face and head.

The term "HLCS" gene, used herein, refers to a gene encoding a holocarboxylase synthetase protein. The protein encoded by the gene plays an important role in effectively using vitamin B (biotin) found in foods such as egg yolk and milk, and is involved in many important cellular functions including production and breakdown of proteins, fats, and carbohydrates.

The term "MX2" gene, used herein, refers to a gene encoding interferon-induced GTP-binding protein Mx2. It is known that the protein encoded by the gene is up-regulated by interferon-alpha, but does not include the antiviral activity of the similar myxovirus resistance protein 1.

The term "MX1" gene, used herein, refers to a gene encoding interferon-induced GTP-binding protein Mx1. Interferon-induced Mx proteins are known to be associated with a specific antiviral state against influenza virus infection in mice.

The term "TMPRSS2" gene, used herein, refers to a gene encoding a transmembrane protease, serine 2 protein. Serine proteases are known to be involved in many physiological and pathological processes, and the gene is known to be up-regulated by androgen hormones in prostate cancer cells and down-regulated in androgen-independent prostate cancer tissues. However, the specific biological function of the gene is unknown.

The term "SLC37A1" gene, used herein, refers to a gene encoding a glucose-6-phosphate exchanger SLC37A1 protein. Unlike a SLC37A4 protein, the protein encoded by the gene does not appear to be involved in blood sugar homeostasis, but is known to regulate phosphate levels in cow's milk and affect the amount of milk produced.

The term "PDE9A" gene, used herein, refers to a gene encoding a high affinity cGMP-specific 3',5'-cyclic phosphodiesterase 9A protein. The protein encoded by the genes is known to play a role in signal transduction by regulating intracellular concentrations of cAMP and cGMP.

The term "CBS" gene, used herein, refers to a gene encoding a cystathionine beta-synthase. Defects in the gene are known to cause cystathionine beta-synthase deficiency (CBSD), resulting in homocystinuria.

The term "CRYAA" gene, used herein, refers to a gene encoding an alpha-crystallin A chain protein. Defects in the gene are known to cause autosomal dominant congenital cataract.

The term "C21orf2" gene, used herein, refers to a gene encoding cilia and flagella associated protein 410 (CFAP410). The gene is known to be associated with retinal dystrophy and spondylometaphyseal dysplasia.

The term "TRPM2" gene, used herein, refers to a gene encoding a transient receptor potential cation channel, subfamily M, member 2 protein. Although the physiological function of the gene is not precisely known, the gene has been reported to be involved in insulin secretion.

The term "TSPEAR" gene, used herein, refers to a gene encoding a thrombospondin type laminin G domain and EAR repeats protein. The gene is known to be related to hearing loss (deafness) and ectodermal dysplasia.

The term "LINC00162" gene, used herein, refers to a P38 inhibited cutaneous squamous cell carcinoma associated LincRNA (PICSAR) gene. The gene is known to be associated with narcolepsy and embryonal testis carcinoma.

The term "SSR4P1" gene, used herein, refers to signal sequence receptor subunit 4 pseudogene 1. The exact function of the gene is not known.

The term "SLC19A1" gene, used herein, refers to a gene encoding a folate transporter 1 protein. It is known that the protein encoded by the gene plays an important role in maintaining the concentration of folic acid in cells.

The term "LOC100129027" gene, used herein, refers to a PCBP3 antisense RNA 1 (PCBP3-AS1) gene. The specific biological function of the gene is unknown.

The term "MCM3AP" gene, used herein, refers to a gene encoding an 80 kDa MCM3-associated protein. The protein encoded by the gene is an MCM3 binding protein, which is known to have a phosphorylation-dependent DNA-primase activity.

The term "YBEY" gene, used herein, refers to a gene encoding a YbeY metalloendoribonuclease. The gene is known to be associated with mesenteric lymphadenitis.

The term "PRMT2" gene, used herein, refers to a gene encoding a protein arginine N-methyltransferase 2. The protein encoded by the gene is known to interact with estrogen receptor alpha.

The term "ITSN1" gene, used herein, refers to a gene encoding an intersectin-1 protein. The gene is known to be associated with vaccinia and schizophrenia 1.

Another aspect provides a use of the second biomarker for diagnosing Down syndrome.

The second biomarker may be at least one selected from the group presented in Table 2 below.

**[Table 2]**

| **No.** | **Gene** | **Location in chromosome** |
|---|---|---|
| 1 | MXRA8 | chr1-1290330-1290333 |
| 2 | MIB2 | chr1-1564776-1564789 |
| 3 | KIF26B | chr1-245851435-245851817 |
| 4 | SP5 | chr2-171573145-171573165 |
| 5 | ZIC4 | chr3-147113849-147113905 |
| 6 | ENPEP, PITX2 | chr4-111532737-111532757 |
| 7 | SH3BP2 | chr4-2800726-2800764 |
| 8 | SEPP1, FLJ32255 | chr5-42950062-42950139 |
| 9 | SHROOM1 | chr5-132158665-132158747 |
| 10 | | chr5-132158828-132158918 |
| 11 | | chr5-132158969-132158984 |
| 12 | LINC00574, LOC154449 | chr6-170338448-170338716 |
| 13 | PRRT4 | chr7-127991715-127991726 |
| 14 | TMEM176B | chr7-150497697-150497702 |
| 15 | MNX1 | chr7-156796810-156796818 |
| 16 | LOC101928483, EGFL7 | chr9-139482774-139512407 |
| 17 | NACC2, C9orf69 | chr9-139001913-139001932 |
| 18 | TLX1 | chr10-102893888-102894961 |
| 19 | FGF8 | chr10-103535480-103535483 |
| 20 | TACC2 | chr10-123923354-123923385 |
| 21 | CPXM2 | chr10-125651146-125651165 |
| 22 | NKX6-2 | chr10-134598133-134598308 |
| 23 | TLX1NB | chr10-102881237-102881254 |
| 24 | IQSEC3 | chr12-187057-187075 |
| 25 | PCDH8 | chr13-53422171-53422174 |
| 26 | F7 | chr13-113764089-113764501 |
| 27 | SOX9 | chr17-70117397-70117415 |
| 28 | PNMAL2 | chr19-46996868-46998096 |
| 29 | THBD | chr20-23028442-23028524 |
| 30 | MAPK8IP2 | chr22-51042807-51042882 |
| 31 | KLHDC7B | chr22-50986907-50987350 |
| 32 | GPR143 | chrX-9733732-9733845 |
| 33 | IGHMBP2, MRGPRD | chr11-68709935-68710848 |

The term "MXRA8" gene, used herein, refers to a gene encoding a matrix remodeling associated 8 protein. The gene or the protein encoded thereby has been known as a biomarker for diagnosing non-muscle invasive bladder cancer (Korean Patent Publication No. 10-2019-0089552).

The term "MIB2" gene, used herein, refers to a gene encoding a mindbomb E3 ubiquitin-protein ligase 2 (MIB2). The protein MIB2 encoded by the gene interacts with actin proteins (alpha 1) and is known to inhibit melanoma invasion.

The term "KIF26B" gene, used herein, refers to a gene encoding a kinesin family member 26B (KIF26B) protein. The protein encoded by the gene is an intracellular motor protein that transports cell organelles along microtubules, and is essential for kidney development, and increased levels of the protein have been observed in some breast and colorectal cancers.

The term "SP5" gene, used herein, means a gene encoding an Sp5 transcription factor. The gene is known to be involved in Wnt-mediated beta catenin signaling and regulation of target gene transcription.

The term "ZIC4" gene, used herein, refers to a gene encoding a zic family member 4 (ZIC4) protein, specifically, a zic family member protein of a C2H2-type zinc finger protein. The protein encoded by the gene is known to be associated with X-linked visceral heterotaxy and holoprosencephaly type 5.

The term "ENPEP" gene, used herein, refers to a gene encoding a glutamyl aminopeptidase. ENPEP is known to be associated with choriocarcinoma and gestational choriocarcinoma.

The term "PITX2" gene, used herein, refers to a gene encoding a protein also known as paired-like homeodomain transcription factor 2, or pituitary homeobox 2. Mutations in the gene are known to be associated with Axenfeld-Rieger syndrome, and iridogoniodysgenesis syndrome.

The term "SH3BP2" gene, used herein, refers to a gene encoding SH3 domain-binding protein 2 (SH3BP2) derived from a gene located on chromosome 4. The protein encoded by this gene is known to be associated with cherubism.

The term "SEPP1" gene, used herein, refers to a gene encoding selenoprotein P. The selenoprotein is an extracellular glycoprotein, which is uncommon in that it contains 9 Sec residues per polypeptide, and is known to act as an antioxidant in the extracellular space.

The term "FLJ32255" gene, used herein, is an uncharacterized LOC643977, which is an RNA gene associated with the IncRNA class.

The term "SHROOM1" gene, used herein, refers to a gene encoding a SHROOM family member 1 (SHROOM1) protein, which plays an important role in the development of the nervous system and other tissues and is involved in microtubule structure during cell elongation. Among the symptoms of Down syndrome, it is involved in congenital heart defects and arthritis.

The term "LINC00574" gene, used herein, means a long intergenic non-protein coding RNA 574, which is an RNA gene associated with the IncRNA class. The LINC00574 gene is known to be associated with breast cancer.

The term "LOC154449" gene, used herein, is an uncharacterized LOC154449, which is an RNA gene associated with the IncRNA class.

The term "PRRT4" gene, used herein, refers to a gene encoding proline rich transmembrane protein 4 (PRRT4), and is known to be associated with Zellweger Syndrome.

The term "TMEM176B" gene, used herein, is a gene encoding transmembrane protein 176B (TMEM176B), and is known to be involved in the maturation process of dendritic cells.

The term "MNX1" gene, used herein, refers to a gene encoding a protein also known as motor neuron and pancreas homeobox 1 (MNX1) protein or homeobox HB9 (HLXB9). Mutations in the gene are known to be associated with Currarino syndrome.

The term "LOC101928483" gene, used herein, refers to a non-coding RNA (ncRNA) and is also referred to as a NOTCH1 associated IncRNA in T cell acute lymphoblastic leukemia 1 (NALT1) gene.

The term "EGFL7" gene, used herein, refers to a gene encoding EGF-like domain-containing protein 7. Expression of the gene is endothelial cell-specific under physiological conditions, but the gene is known to be aberrantly expressed by tumor cells in human cancer.

The term "NACC2" gene, used herein, means a gene encoding an NACC family member 2 protein. The protein encoded by the gene is known to be associated with lateral myocardial infarction and interstitial myocarditis.

The term "C9orf69" gene, used herein, means a gene encoding transmembrane protein 250, and is also called TMEM250. It is known that the protein encoded by the gene is capable of playing an important role in cell proliferation by promoting progression to the S phase in the cell cycle.

The term "TLX1" gene, used herein, refers to a gene encoding T-cell leukemia homeobox protein 1 (TLX1), and is also called HOX11. The protein encoded by the gene is known to interact with serine/threonine-protein phosphatase PP1-gamma catalytic subunit (PPP1CC), serine/threonine-protein phosphatase 2A catalytic subunit beta isoform (PPP2CB), and serine/threonine-protein phosphatase 2A catalytic subunit alpha isoform (PPP2CA).

The term "FGF8" gene, used herein, refers to a gene encoding a fibroblast growth factor 8 (FGF8) protein. The protein encoded by the gene supports androgen- and anchorage-independent growth of mammary tumor cells, and overexpression of this gene is known to increase tumor growth and angiogenesis.

The term "TACC2" gene, used herein, refers to a gene encoding transforming acidic coiled-coil-containing protein 2 (TACC2). The gene encodes a protein that is accumulated at the centrosome throughout the cell cycle, and the gene is present in chromosomal regions associated with tumorigenesis. Expression of the gene is known to affect progression of breast tumors.

The term "CPXM2" gene, used herein, refers to a gene encoding a carboxypeptidase X, M14 family member 2 (CPXM2) protein.

The term "NKX6-2" gene, used herein, refers to a gene encoding an NK6 homeobox 2 (NKX6-2) protein. The protein encoded by the gene is known to be associated with spastic ataxia and autosomal recessive disease.

The term "TLX1NB" gene, used herein, refers to a TLX1 Neighbor (TLX1NB) RNA gene and belongs to the IncRNA class.

The term "IQSEC3" gene, used herein, refers to a human gene known as IQ motif and Sec7 domain 3, and is also called KIAA1110. It is known that the gene is highly expressed in the brain, particularly in the amygdala, and plays an important role in learning.

The term "PCDH8" gene, used herein, refers to a gene encoding a protocadherin-8 (PCDH8) protein. The gene encodes an endogenous membrane protein that is thought to function in cell adhesion in a central nervous system (CNS)-specific manner.

The term "F7" gene, used herein, refers to a gene encoding coagulation factor VII, a vitamin K-dependent factor essential for hemostasis. The gene is known to be associated with factor VII deficiency and myocardial infarction.

The term "SOX9" gene, used herein, means a gene encoding the transcription factor SOX-9 protein. Mutations in this gene are known to be associated with skeletal malformation syndrome and campomelic dysplasia.

The term "PNMAL2" gene, used herein, refers to a gene encoding a PNMA family member 8B protein, and an important paralog of the gene is PNMA8A. The protein encoded by the gene is paraneoplastic antigen-like protein 8B.

The term "THBD" gene, used herein, refers to a gene encoding a thrombomodulin protein. The protein encoded by the gene is a protein derived from endothelial cells of blood vessels and serves to prevent generation of blood clots, in cooperation with other factors.

The term "MAPK8IP2" gene, used herein, refers to a gene encoding C-jun-amino-terminal kinase-interacting protein 2, and is also called islet-brain-2 (IB2). It is known that the protein encoded by the gene is highly expressed in the brain and is almost always lacking in Phelan-McDermid syndrome.

The term "KLHDC7B" gene, used herein, refers to a gene encoding a kelch domain containing 7B protein. This gene is known to be associated with chlamydia pneumonia.

The term "GPR143" gene, used herein, refers to a gene encoding a G-protein coupled receptor 143 (GPR143) protein. The gene is known to be regulated by microphthalmia-associated transcription factors.

The term "IGHMBP2", used herein, refers a gene that encodes immunoglobulin helicase µ-binding protein 2 (IGHMBP2), cardiac transcription factor 1 (CATF1), or a protein known as DNA-binding protein SMUBP-2. Mutations in the gene are known to cause distal spinal muscular atrophy type 1.

The term "MRGPRD" gene, used herein, refers to a gene encoding a Mas-related G-protein coupled receptor member D protein. The gene is known to be associated with femoral cancer and liver rhabdomyosarcoma.

By comparing a methylation level of the first biomarker or a methylation level of the second biomarker of a fetus with a methylation level of a normal control group, the presence or risk of Down syndrome in the fetus may be determined.

Among the first biomarkers, the CHODL, NCAM2, CYYR1, GRIK1, OLIG2, CLIC6, SIM2, HLCS, MX2, MX1, TMPRSS2, SLC37A1, PDE9A, CBS, CRYAA, C21orf2, TRPM2, TSPEAR, LINC00162, SSR4P1, SLC19A1, LOC100129027, MCM3AP, YBEY, and PRMT genes may be hyper-methylated in Down syndrome fetal placentas compared to blood of mothers pregnant with normal fetuses or normal fetal placentas; and the ITSN1 gene may be hypo-methylated.

Among the second biomarkers, the MXRA8, MIB2, KIF26B, SP5, ZIC4, ENPEP, PITX2, SH3BP2, SEPP1, FLJ32255, SHROOM1, LINC00574, LOC154449, PRRT4, TMEM176B, MNX1, LOC101928483, EGFL7, NACC2, C9orf69, TLX1, FGF8, TACC2, CPXM2, NKX6-2, TLX1NB, IQSEC3, PCDH8, F7, SOX9, PNMAL2, THBD, MAPK8IP2, KLHDC7B, and GPR143 genes may be hyper-methylated in Down syndrome fetal placentas compared to blood of mothers pregnant with normal fetuses or normal fetal placentas; and the IGHMBP2, and MRGPRD genes may be hypo-methylated.

The term "hyper-methylation", used herein, may refer to a state in which the methylation level of the experimental group is higher than that of the control group as a result of measuring the methylation level. The term "hypo-methylation", used herein, may refer to a state in which the methylation level of the experimental group is lower than that of the control group as a result of measuring the methylation level.

When a methylation level of the first biomarker measured in a fetus is hypo-methylated or hyper-methylated by 10 % to 30 % or more, compared to the methylation level measured in the blood of mothers pregnant with a normal fetus or in normal fetal placentas, the fetus may be determined to have or be at a high risk of having Down syndrome. In an embodiment, the methylation level of the first biomarker measured in normal fetal placentas was confirmed to be 10 % to 20 % or more hypo-methylated or hyper-methylated compared to the methylation level measured in normal maternal blood, and the first biomarker was confirmed to be a tissue (placenta)-specific marker. In addition, in an embodiment, the methylation level of the first biomarker measured in Down syndrome fetal placentas was confirmed to be 10 % to 20 % or more hypo-methylated or hyper-methylated compared to the methylation level measured in normal fetal placentas, and the first biomarker was confirmed to be a disease (Down syndrome)-specific marker.

When a methylation level of the second biomarker measured in a fetus is hypo-methylated or hyper-methylated by 30 % to 50 % or more, compared to the methylation level measured in the blood of mothers pregnant with a normal fetus or in normal fetal placentas, the fetus may be determined to have or be at a high risk of having Down syndrome. In an embodiment, the methylation level of the first biomarker measured in normal fetal placentas was confirmed to be 10 % to 20 % or more hypo-methylated or hyper-methylated compared to the methylation level measured in normal maternal blood, and the second biomarker was confirmed to be a tissue (placenta)-specific marker. In addition, in an embodiment, the methylation level of the second biomarker measured in Down syndrome fetal placentas was confirmed to be 30 % to 50 % or more hypo-methylated or hyper-methylated compared to the methylation level measured in normal fetal placentas, and the first biomarker was confirmed to be a disease (Down syndrome)-specific marker.

The term "biological sample", used herein, may include samples such as tissues, cells, whole blood, serum, plasma, saliva, sputum, cerebrospinal fluid, and urine isolated from a fetus, and may include cell-free DNA, which is DNA free in the blood and is not present in the cell nucleus. The biological sample may be derived from the placenta.

The term "placenta" refers to a structure that mediates material exchange between a fetus and the mother necessary for the growth and survival of the fetus, and is formed when a part of the fetal membrane surrounding the fetus adheres to the mother's endometrium. The placenta may include chorion. The term "chorion" corresponds to the middle layer among the decidua, chorion, and amnion, which are membranes that enclose amniotic fluid in the uterus. The chorion develops from the fertilized egg and forms part of the egg membrane, and the chorionic villi, which are myriad protrusions on the front side, grow densely and play a role in invading the fertilized egg into the inner wall of the uterus. For example, the biological sample may be derived from chorionic villus cells. Preferably, the biological sample may refer to cell-free DNA in maternal blood derived from chorionic villus cells.

One aspect provides a method of diagnosing Down syndrome including: measuring a methylation level of a Down syndrome biomarker in a biological sample separated from a subject, wherein the biomarker is a first biomarker present on chromosome 21 or a combination with a second biomarker present on a chromosome other than chromosome 21; comparing the measured methylation level of the biomarker with a methylation level of the first biomarker and the second biomarker in the biological sample separated from a normal control group; and determining a presence or a risk of Down syndrome by comparing the methylation levels.

Details of each process of the diagnostic method are the same as those described in the method of providing information for diagnosing Down syndrome.

### Advantageous Effects

The method according to an aspect has an effect of efficiently diagnosing Down syndrome by simple measurement of gene methylation levels. In addition, by comparing the measured methylation levels with the methylation level of biomarkers measured in a normal control group (for example, blood of mothers pregnant with a normal fetus or normal fetal placentas), a presence or risk of Down syndrome in the fetus may be diagnosed.

### Description of Drawings

FIG. 1 shows results of comparing average methylation levels among 65 biomarker groups through analysis of methylation levels of chromosome 21 for normal maternal blood (NB), normal fetal placental tissue (NT), and Down syndrome fetal placental tissue (T21T).
FIG. 2 shows results of comparing methylation levels among 33 biomarker groups through analysis of methylation levels of a genome for the normal maternal blood (NB), the normal fetal placental tissue (NT), and the Down syndrome fetal placental tissue (T21T).

### Mode for Invention

Hereinafter, the present disclosure will be described in more detail through examples. However, these examples are intended to illustrate the present disclosure, and the scope of the present disclosure is not limited to these examples.

### Example 1: DNA Isolation from maternal blood and placental tissue

The experiment was conducted under approval of the Institutional Review Board of Cheil hospital (# CGH-IRB-2016-5). This experiment was conducted on pregnant women with singleton pregnancy who received medical cares in the department of obstetrics and gynecology at Cheil Hospital from June 2015 to May 2017. Written informed consent was obtained from all patients with IRB approval.

The placental tissue was chorionic villus cells used for chorionic villus tests which are harvested in early pregnancy (12 to 13 weeks of gestation) and stored in liquid nitrogen until analysis. The gestational age of each fetus was determined by ultrasonography. A chromosome analysis using the Giemsa-trypsin-Giemsa (GTG) banding method was performed to determine the karyotype of fetal chorionic villus cells. All the placental tissues of the Down syndrome (DS) group had a full extra copy of HSA21 (47, XX, +21, or 47, XY, +21), and all the placental tissues of the control group (normal, N) exhibited a normal karyotype (46, XX, or 46, XY). The sex ratio of fetuses was matched between the experimental group (Down syndrome fetus group) and the control group (normal fetus group). Genomic DNA of each sample was extracted from chorionic villus cells by using the QIAamp DNA mini kit (Qiagen, catalog number 51304) according to the manufacturer's instructions. Maternal blood was collected prior to chorionic villus testing and genomic DNA was immediately extracted by using the QIAamp DNA mini kit (Qiagen, catalog number 51304) according to the manufacturer's instructions and stored in a cryogenic freezer until analyzed.

As shown in Table 3 below, there was no significant difference between the experimental group and the control group in relation to the maternal age, gestational age, translucency, and fetal sex ratio in the collection of chorionic villus cells (P> 0.05).

**[Table 3]**

| **Nu mb er** | **Karyotype** | **Gestational age (weeks)** | **Sex of the fetus** | **Maternal age** | **Nuchal translucency (mm)** |
|---|---|---|---|---|---|
| N1 | 46, XY | 12+1 | Male | 35 | 4.4 |
| N2 | 46, XY | 12+1 | Male | 38 | 1.9 |
| N3 | 46, XX | 12+6 | Female | 39 | 2.5 |
| N4 | 46, XY | 12+1 | Male | 37 | 2.8 |
| N5 | 46, XX | 12+1 | Female | 37 | 2.8 |
| D1 | 47, XX, +21 | 12+4 | Female | 35 | 3.9 |
| D2 | 47, XY, +21 | 12+1 | Male | 39 | 2.9 |
| D3 | 47, XY, +21 | 12+3 | Male | 39 | 4.6 |
| D4 | 47, XX, +21 | 12+1 | Female | 41 | 4.9 |
| D5 | 47, XY, +21 | 13+1 | Male | 33 | 9.1 |

### Example 2: High efficiency methylome profiling by using MC-seq

Based on the DNA samples of the normal maternal blood, the normal fetal placental tissues, and the Down syndrome placental tissues collected in Example 1, DNA methylation was quantified at various CpG sites by using MC-seq, and methylome profiling was performed.

A standard DNA methylation region capture library was generated by using the SureSelect Methyl-Seq Target Enrichment protocol (Agilent) for paired-end sequencing libraries (ver. B.3, June 2015; Illumina) with 3 µg of genomic DNA. A SureSelect Human Methyl-Seq probe set (Agilent, catalog number 5190-4662) was used. Quantification of DNA and quality assessment of DNA were performed by measuring with a PicoGreen assay kit (Thermo Fisher Scientific, catalog number P7589) and a Nanodrop spectrophotometer (NanoDrop Technologies, catalog number ND-2000), respectively. 3 µg of the genome was fragmented to a target size of 150 bp to 200 bp of DNA by using an ultrasonicator (AFA; Covaris, catalog number 500219). Briefly, 8 microtube strips were loaded onto the tube holder of the sonicator and DNA was sheared by using the following setting: mode, frequency sweeping; duty cycle, 10 %; intensity, 5; cycles per burst, 200; duration, 60 sec × 6 cycles; temperature, 4 °C to 7 °C. The fragmented DNAs were repaired, 'A' was ligated to the 3' end, and SureSelect Methyl-Seq Methylated Adapter was ligated to the fragment. After the ligation was evaluated, the adapter-ligated products were amplified by PCR.

Subsequently, the final purified product, such as methylated adapter-ligated DNA was then quantified according to the qPCR quantification protocol guide and verified by using a TapeStation DNA screen tape D1000 (Agilent, catalog number 5067-5582). For DNA methylation region capture, 350 ng of DNA library was mixed with hybridization buffer, blocking mix, RNase block, and 5 µl of SureSelect All DNA methylation region capture library according to the standard SureSelect Methyl-Seq Target Enrichment protocol (Agilent). Hybridization with capture baits was performed at 65 °C by using a PCR machine with a thermal cycler read option heated at 105 °C for 24 hours. The target captured DNA was treated with bisulfite by using a EZ DNA Methylation-Gold Kit (Zymo Research, catalog number D5005), and 8 PCR cycles to enrich the adapter-added fragments and 6 PCR cycles to add multiplexing barcodes were performed. The captured DNA was amplified. The final purified product was quantified by using the qPCR quantification protocol guide mentioned above and verified by using the TapeStation DNA screen tape D1000 (Agilent, catalog number 5067-582). Sequencing was performed by using the HiSeq^{™} 2500 platform (Illumina, catalog number SY-401-2501).

### Example 3: Data processing and methylation profiling

Data processing and methylation profiling analysis were performed for the final product methylated in Example 2.

The quality of the paired end raw reads generated from the sequencing was identified by using a FastQC software (version 0.11.5). Before starting the analysis, Trimmomatic (version 0.32) was used to remove adapter sequences and bases with a base quality of 3 or less from the final data. In addition, the sliding window trim method was used to remove bases that did not satisfy window size = 4 and average quality = 15. Data with a minimum length of 36 bp were removed to generate organized data. The washed reads were aligned to the Homo sapiens genome (UCSC hg19) by using a bisulfite sequencing MAPping program (BSMAP; version 2.90 parameters set-n 1 -r 0) based on an unidirectional short oligonucleotide alignment program (SOAP), and the washed reads could be uniquely mapped in the data. The mapped data in a SAM file format were aligned and indexed by using SAMtools (version 1.2). PCR duplicates were removed with sambamba (version 0.5.9). Methylation levels were measured with the metratio.py feature in the BSMAP program. A methylation ratio higher than the 10 CT number of all single cytosines located in the Agilent SureSelect target region may indicate that general methylation is completed. For regions covered by both ends of a read pair, only one read was used to call methylation. The profiles within the range the results are applied were summarized as follows: # of C / actual CT number for each of the three sequence contexts (CG, CHG, and CHH).

After reading the methylation level, the methylation level at each base of CpG was normalized with intermediate scaling normalization to distinguish between DMCs and DMRs. For five comparison pairs, an independent T-test was used to assess significance of differences of the methylation between the two groups. For the P value, false discovery results were controlled in multiple tests by using the Benjamini and Hochberg false discovery rate (FDR) method, and correcting. An analysis of main components showed segregation of the samples based on the disease status (normal or DS) as in previous studies, but not on fetal sex. DMC was determined by filtering out each region associated with |delta_mean| ≥ 0.1, independent T-test p-value < 0.05, and FDR < 0.05. DMR was defined as a contiguous region of any length containing ≥ 3 DMCs. A hierarchical clustering analysis was also performed by using complete linkage and Euclidean distance as measures of similarity for indicating methylation levels of samples for significant CpGs that satisfy one or more comparison pairs. Heatmaps were automatically plotted by centroid linkage by using the centroid absolute correlation of similarity metric. All data analysis and visualization of differentially methylated results were performed by using R 3.3.1 (www.r-project.org) and Statistical Package for Social Sciences 12.0 (SPSS Inc.).

### Example 4: Discovery of specific epigenetic markers in Down syndrome fetal placentas

Genomic methylation patterns were comparatively analyzed at a CpG site level by using the method of Example 2, for 5 samples each of blood of mothers with normal fetuses, normal fetal placentas, and Down syndrome fetal placentas obtained in Example 1. In this regard, the methylation level (%) of each CpG site was expressed in a scale of 0 to 100, with 0 being unmethylated and 100 being completely methylated. Depending on the difference in methylation levels that was comparatively analyzed, hyper-methylated or hypo-methylated biomarkers were discovered.

### 4-1. Comparison of methylation levels on chromosome 21

The CHODL, NCAM2, CYYR1, GRIK1, OLIG2, CLIC6, SIM2, HLCS, MX2, MX1, TMPRSS2, SLC37A1, PDE9A, CBS, CRYAA, TRPM2, C21orf2, TSPEAR, LINC00162, SSR4P1, SLC19A1, LOC100129027, MCM3AP, YBEY, PRMT2, and ITSN1 gene regions were selected as regions with three or more consecutive epigenetic characteristics of the same type from chromosome 21, the target chromosome of Down syndrome. Detailed information of the selected 65 gene regions is shown in Table 4 below.

**[Table 4]**

| **Gene symbol** | **Region** | | | |
|---|---|---|---|---|
| | **Functional part** | **bp** | **Number of CGs** | **Seq.** |
| CHODL | UTR5 | 72 | 6 | chr21-19617176-19617247 |
| | | 71 | 13 | chr21-19617336-19617406 |
| | intronic | 80 | 9 | chr21-19617673-19617752 |
| NCAM2 | upstream | 36 | 5 | chr21-22370043-22370078 |
| CYYR1 | intronic | 176 | 4 | chr21-27944498-27944673 |
| GRIK1 | intronic | 52 | 3 | chr21-31311263-31311314 |
| | | 125 | 11 | chr21-31311387-31311511 |
| OLIG2 | intergenic | 235 | 14 | chr21-34391993-34392227 |
| | | 17 | 4 | chr21-34392448-34392476 |
| | | 173 | 7 | chr21-34392696-34392868 |
| | | 42 | 4 | chr21-34395135-34395176 |
| | | 75 | 5 | chr21-34395490-34395566 |
| | | 90 | 6 | chr21-34395876-34395965 |
| | | 50 | 3 | chr21-34396297-34396346 |
| | | 269 | 16 | chr21-34396460-34396728 |
| | | 117 | 9 | chr21-34397003-34397119 |
| | exonic | 71 | 12 | chr21-34399292-34399362 |
| | | 103 | 12 | chr21-34399427-34399529 |
| | | 34 | 4 | chr21-34399681-34399714 |
| | | 56 | 8 | chr21-34399848-34399903 |
| | UTR3 | 68 | 10 | chr21-34400145-34400212 |
| | | 275 | 9 | chr21-34400714-34400988 |
| | | 14 | 3 | chr21-34401366-34401379 |
| | downstream | 236 | 11 | chr21-34401534-34401769 |
| CLIC6 | exonic | 32 | 6 | chr21-36042254-36042285 |
| | | 203 | 25 | chr21-36042533-36042735 |
| SIM2 | intergenic | 81 | 4 | chr21-38067964-38068044 |
| | | 389 | 29 | chr21-38068418-38068806 |
| | | 173 | 15 | chr21-38069018-38069190 |
| | | 1206 | 87 | chr21-38069475-38070680 |
| | intronic | 13 | 3 | chr21-38072494-38072506 |
| | | 44 | 5 | chr21-38073032-38073075 |
| | | 66 | 8 | chr21-38073463-38073528 |
| | | 57 | 3 | chr21-38073992-38074050 |
| | | 31 | 3 | chr21-38074152-38074184 |
| | | 181 | 6 | chr21-38074992-38075172 |
| | | 343 | 30 | chr21-38076769-38077111 |
| | | 193 | 12 | chr21-38077206-38077398 |
| | | 197 | 8 | chr21-38078262-38078458 |
| | | 232 | 7 | chr21-38079128-38079359 |
| | | 39 | 4 | chr21-38079924-38079962 |
| | | 191 | 18 | chr21-38079988-38080178 |
| | | 55 | 7 | chr21-38081200-38081254 |
| | | 62 | 7 | chr21-38081394-38081455 |
| | | 17 | 4 | chr21-38083112-38083128 |
| HLCS | intronic | 76 | 8 | chr21-38352985-38353060 |
| | UTR5 | 65 | 4 | chr21-38353193-38353257 |
| MX2 | intronic | 177 | 14 | chr21-42741677-42741853 |
| MX1 | intronic | 154 | 13 | chr21-42798695-42798848 |
| TMPRSS2 | intronic | 90 | 3 | chr21-42878480-42878569 |
| SLC37A1, PDE9A | intergenic | 46 | 3 | chr21-44011221-44011266 |
| CBS | UTR3 | 97 | 4 | chr21-44473401-44473497 |
| CRYAA | upstream | 137 | 13 | chr21-44588388-44588457 |
| C21orf2, TRPM2 | intergenic | 88 | 12 | chr21-45769923-45770010 |
| TSPEAR | intronic | 167 | 8 | chr21-46126022-46126188 |
| | | 210 | 17 | chr21-46126891-46127100 |
| | | 179 | 10 | chr21-46128800-46128978 |
| | | 531 | 39 | chr21-46129159-46129689 |
| LINC00162, SSR4P1 | intergenic | 27 | 3 | chr21-46439211-46439237 |
| SLC19A1,LOC100129 027 | intergenic | 23 | 3 | chr21-47062136-47062158 |
| MCM3AP | exonic | 33 | 4 | chr21-47704178-47704210 |
| YBEY | downstream | 25 | 4 | chr21-47717843-47717867 |
| | | 93 | 13 | chr21-47717931-47718023 |
| PRMT2, NONE | intergenic | 76 | 11 | chr21-48087183-48087258 |
| ITSN1 | intronic | 63 | 4 | chr21-35246628-35246690 |

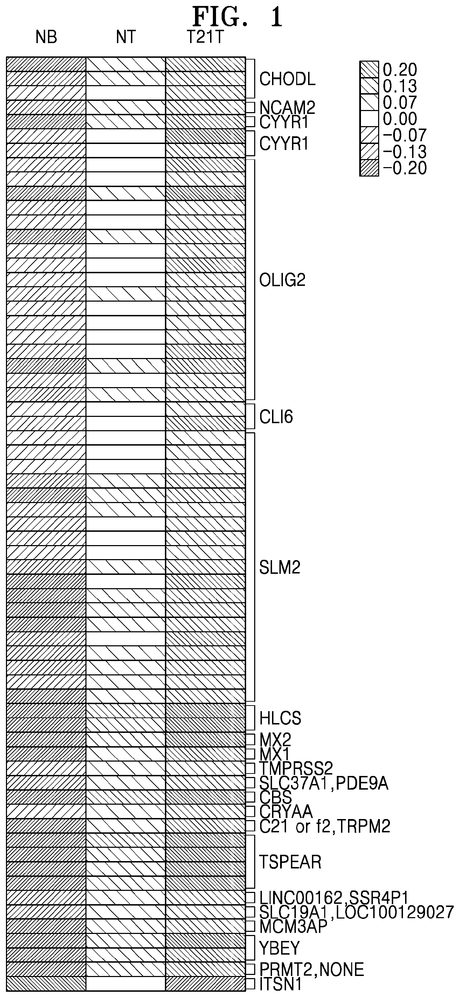

The degrees of methylation (the value obtained by dividing the methylation level (%) by 100) of the selected gene regions in normal maternal blood, normal fetal placentas, and Down syndrome fetal placentas are shown in Table 5 below and FIG. 1.

**[Table 5]**

| Epigenetic Characteristics | Gene symbol | Methylation degree | | |
|---|---|---|---|---|
| | | Normal maternal blood | Normal fetal placenta | Down syndrome fetal placenta |
| Hyper-methylation | CHODL | 0.05 | 0.31 | 0.44 |
| | | 0.04 | 0.26 | 0.37 |
| | | 0.01 | 0.15 | 0.30 |
| | NCAM2 | 0.02 | 0.23 | 0.34 |
| | CYYR1 | 0.04 | 0.29 | 0.39 |
| | GRIK1 | 0.14 | 0.29 | 0.46 |
| | | 0.09 | 0.27 | 0.40 |
| | OLIG2 | 0.02 | 0.18 | 0.34 |
| | | 0.03 | 0.15 | 0.29 |
| | | 0.03 | 0.28 | 0.43 |
| | | 0.03 | 0.19 | 0.31 |
| | | 0.02 | 0.12 | 0.25 |
| | | 0.02 | 0.23 | 0.34 |
| | | 0.04 | 0.18 | 0.31 |
| | | 0.11 | 0.24 | 0.40 |
| | | 0.10 | 0.23 | 0.38 |
| | | 0.06 | 0.25 | 0.36 |
| | | 0.06 | 0.19 | 0.32 |
| | | 0.04 | 0.20 | 0.30 |
| | | 0.03 | 0.13 | 0.27 |
| | | 0.06 | 0.21 | 0.37 |
| | | 0.06 | 0.28 | 0.40 |
| | | 0.03 | 0.20 | 0.31 |
| | | 0.04 | 0.24 | 0.34 |
| Hyper-methylation | CLIC6 | 0.01 | 0.13 | 0.24 |
| | | 0.02 | 0.16 | 0.33 |
| | SIM2 | 0.12 | 0.22 | 0.34 |
| | | 0.11 | 0.25 | 0.35 |
| | | 0.08 | 0.19 | 0.29 |
| | | 0.05 | 0.26 | 0.38 |
| | | 0.04 | 0.30 | 0.40 |
| | | 0.03 | 0.22 | 0.32 |
| | | 0.04 | 0.22 | 0.33 |
| | | 0.04 | 0.20 | 0.32 |
| | | 0.04 | 0.19 | 0.30 |
| | | 0.12 | 0.32 | 0.45 |
| | | 0.04 | 0.23 | 0.39 |
| | | 0.08 | 0.31 | 0.42 |
| | | 0.11 | 0.34 | 0.44 |
| | | 0.09 | 0.33 | 0.44 |
| | | 0.09 | 0.31 | 0.45 |
| | | 0.08 | 0.30 | 0.43 |
| | | 0.04 | 0.25 | 0.37 |
| | | 0.04 | 0.24 | 0.35 |
| | | 0.06 | 0.29 | 0.39 |
| | | 0.04 | 0.57 | 0.71 |
| | HLCS | 0.03 | 0.50 | 0.66 |
| | MX2 | 0.26 | 0.62 | 0.74 |
| | MX1 | 0.05 | 0.30 | 0.45 |
| | TMPRSS2 | 0.33 | 0.54 | 0.64 |
| | SLC37A1, PDE9A | 0.08 | 0.31 | 0.42 |
| Hyper-methylation | CBS | 0.21 | 0.67 | 0.77 |
| | CRYAA | 0.43 | 0.61 | 0.71 |
| | C21orf2, TRPM2 | 0.15 | 0.41 | 0.54 |
| | TSPEAR | 0.12 | 0.60 | 0.73 |
| | | 0.16 | 0.57 | 0.72 |
| | | 0.09 | 0.46 | 0.62 |
| | | 0.05 | 0.46 | 0.62 |
| | LINC00162, SSR4P1 | 0.23 | 0.48 | 0.59 |
| | SLC19A1, LOC100129027 | 0.24 | 0.46 | 0.57 |
| | MCM3AP | 0.16 | 0.44 | 0.55 |
| | YBEY | 0.06 | 0.49 | 0.67 |
| | | 0.11 | 0.48 | 0.64 |
| | PRMT2,NONE | 0.01 | 0.34 | 0.44 |
| Hypo-methylation | ITSN1 | 0.92 | 0.66 | 0.36 |

A difference in methylation levels between normal fetal placentas and maternal blood cells, and a difference in methylation levels between Down syndrome fetal placentas and normal fetal placentas were compared.

As a result, as shown in Table 3 and FIG. 1, the CHODL, NCAM2, CYYR1, GRIK1, OLIG2, CLIC6, SIM2, HLCS, MX2, MX1, TMPRSS2, SLC37A1, PDE9A, CBS, CRYAA, TRPM2, C21orf2, TSPEAR, SSR4P1, LINC00162, MCM3AP, YBEY, and PRMT2 (NONE) gene regions were confirmed to be hyper-methylated in the fetal placentas, especially in the Down syndrome fetal placentas. Specifically, the difference in methylation levels of the genes between the normal fetal placentas and maternal blood cells was 10 to 55, and the genes were hyper-methylated in the fetal placentas compared to the maternal blood, and thus, the genes were confirmed to be tissue (placenta)-specific biomarkers. In addition, the difference in methylation levels of the genes between the Down syndrome fetal placentas and normal fetal placentas was 10 to 20, confirming that the genes are disease (Down syndrome)-specific biomarkers hyper-methylated in a Down syndrome fetus compared to a normal fetus. Differences in the methylation levels among the Down syndrome fetal placentas and the other two groups (normal fetal placentas and maternal blood) were all statistically significant (P < 0.05).

In addition, it was confirmed that the ITSN1 gene region was hypo-methylated in the Down syndrome fetal placentas. Specifically, it was confirmed that the methylation level of the ITSN1 gene region in maternal blood cells was 90 or more, and the methylation level in the normal fetal placentas was 65 or more, and hyper-methylated, whereas the methylation level in the Down syndrome fetal placentas was 40 or less, and hypo-methylated. The difference in methylation levels between the groups was 25 or more. Even in this case, differences in the methylation levels among the Down syndrome fetal placentas and the other two groups (normal fetal placentas and maternal blood) were all statistically significant (P < 0.05).

### 4-2. Comparison of methylation levels in other chromosomes, except for chromosome 21

The MXRA8, MIB2, KIF26B, SP5, ZIC4, ENPEP, PITX2, SH3BP2, SEPP1, FLJ32255, SHROOM1, LINC00574, LOC154449, PRRT4, TMEM176B, MNX1, LOC101928483, EGFL7, NACC2, C9orf69, TLX1, FGF8, TACC2, CPXM2, NKX6-2, TLX1NB, IQSEC3, PCDH8, F7, SOX9, PNMAL2, THBD, MAPK8IP2, KLHDC7B, GPR143, IGHMBP2, and MRGPRD gene regions were selected as regions with two or more consecutive epigenetic characteristics of the same type. Detailed information of the selected 33 gene regions is shown in Table 6 below.

**[Table 6]**

| **Chro moso me** | **Gene symbol** | **Region** | | |
|---|---|---|---|---|
| | | **Functional part** | **bp** | **Seq.** |
| 1 | MXRA8 | exonic | 4 | chr1-1290330-1290333 |
| 1 | MIB2 | exonic | 14 | chr1-1564776-1564789 |
| 1 | KIF26B | exonic | 383 | chr1-245851435-245851817 |
| 2 | SP5 | exonic | 21 | chr2-171573145-171573165 |
| 3 | ZIC4 | exonic | 57 | chr3-147113849-147113905 |
| 4 | ENPEP, PITX2 | intergenic | 21 | chr4-111532737-111532757 |
| 4 | SH3BP2 | intronic | 39 | chr4-2800726-2800764 |
| 5 | SEPP1,FLJ32255 | intergenic | 78 | chr5-42950062-42950139 |
| 5 | SHROOM1 | exonic | 83 | chr5-132158665-132158747 |
| | | intronic | 91 | chr5-132158828-132158918 |
| | | exonic | 16 | chr5-132158969-132158984 |
| 6 | LINC00574, LOC154449 | intergenic | 269 | chr6-170338448-170338716 |
| 7 | PRRT4 | exonic | 12 | chr7-127991715-127991726 |
| 7 | TMEM176B | intronic | 6 | chr7-150497697-150497702 |
| 7 | MNX1 | downstream | 9 | chr7-156796810-156796818 |
| 9 | LOC 101928483, EGFL7 | intergenic | 29634 | chr9-139482774-139512407 |
| 9 | NACC2, C9orf69 | intergenic | 20 | chr9-139001913-139001932 |
| 10 | TLX1 | intronic | 1074 | chr10-102893888-102894961 |
| 10 | FGF8 | intronic | 4 | chr10-103535480-103535483 |
| 10 | TACC2 | UTR5 | 32 | chr10-123923354-123923385 |
| 10 | CPXM2 | exonic | 20 | chr10-125651146-125651165 |
| 10 | NKX6-2 | downstream | 176 | chr10-134598133-134598308 |
| 10 | | intronic | 18 | chr10-102881237-102881254 |
| 12 | IQSEC3 | intronic | 19 | chr12-187057-187075 |
| 13 | PCDH8 | exonic | 4 | chr13-53422171-53422174 |
| 13 | F7 | intronic | 413 | chr13-113764089-113764501 |
| 17 | SOX9 | UTR5 | 19 | chr17-70117397-70117415 |
| 19 | PNMAL2 | exonic | 1237 | chr19-46996868-46998096 |
| 20 | THBD | exonic | 83 | chr20-23028442-23028524 |
| 22 | MAPK8IP2 | exonic | 76 | chr22-51042807-51042882 |
| 22 | KLHDC7B | exonic | 444 | chr22-50986907-50987350 |
| X | GPR143 | exonic | 114 | chrX-9733732-9733845 |
| 11 | IGHMBP2, MRGPRD | intergenic | 914 | chr11-68709935-68710848 |

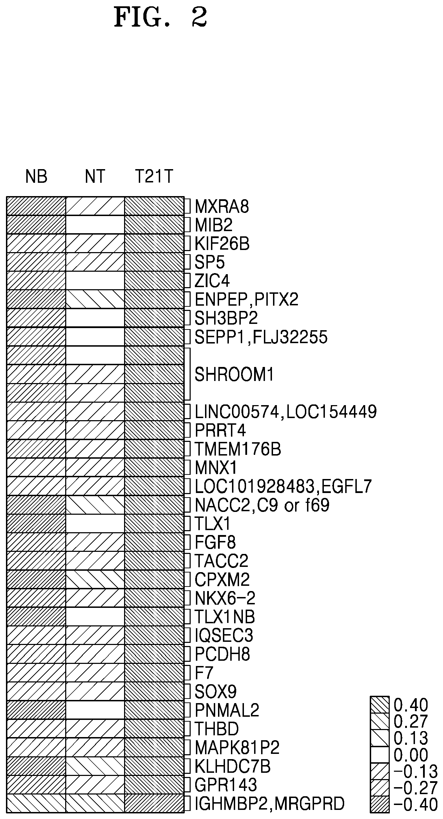

The degrees of methylation (the value obtained by dividing the methylation level (%) by 100) of the selected gene regions in the normal maternal blood, normal fetal placentas, and Down syndrome fetal placentas are shown in Table 7 below and FIG. 2.

**[Table 7]**

| Epigenetic Characteristics | Chromosome | Gene | Methylation degree | | |
|---|---|---|---|---|---|
| | | | Normal maternal blood | Normal fetal placenta | Down syndrome fetal placenta |
| Hyper-methylation | 1 | MXRA8 | 0.12 | 0.42 | 0.78 |
| | 1 | MIB2 | 0.17 | 0.49 | 0.85 |
| | 1 | KIF268 | 0.16 | 0.37 | 0.79 |
| | 2 | SPS | 0.08 | 0.31 | 0.79 |
| | 3 | ZIC4 | 0.00 | 0.29 | 0.68 |
| | 4 | ENPEP, PITX2 | 0.03 | 0.45 | 0.74 |
| | 4 | SH3BP2 | 0.02 | 0.33 | 0.71 |
| | 5 | SEPP1, FLI32255 | 0.01 | 0.33 | 0.70 |
| | 5 | SHROOM1 | 0.08 | 0.40 | 0.77 |
| | | | 0.05 | 0.30 | 0.70 |
| | | | 0.07 | 0.34 | 0.73 |
| | 6 | LINC00574, LOC154449 | 0.13 | 0.34 | 0.77 |
| | 7 | PRRT4 | 0.09 | 0.19 | 0.67 |
| | 7 | TMEM176B | 0.03 | 0.29 | 0.73 |
| | 7 | MNX1 | 0.00 | 0.12 | 0.61 |
| | 9 | LOC101928483, EGFL7 | 0.21 | 0.31 | 0.80 |
| | 9 | NACC2, C9orf69 | 0.00 | 0.39 | 0.70 |
| Hyper-methylation | 10 | TLX1 | 0.02 | 0.36 | 0.72 |
| | 10 | FGF8 | 0.03 | 0.22 | 0.70 |
| | 10 | TACC2 | 0.02 | 0.26 | 0.66 |
| | 10 | CPXM2 | 0.01 | 0.51 | 0.79 |
| | 10 | NKX6-2 | 0.05 | 0.28 | 0.69 |
| | 10 | TLX1NB | 0.01 | 0.36 | 0.71 |
| | 12 | IQSEC3 | 0.03 | 0.18 | 0.64 |
| | 13 | PCDH8 | 0.10 | 0.31 | 0.77 |
| | 13 | F7 | 0.06 | 0.24 | 0.68 |
| | 17 | SOX9 | 0.03 | 0.25 | 0.64 |
| | 19 | PNMAL2 | 0.03 | 0.42 | 0.78 |
| | 20 | THBD | 0.21 | 0.39 | 0.83 |
| | 22 | MAPK8IP2 | 0.14 | 0.27 | 0.73 |
| | 22 | KLHDC78 | 0.03 | 0.55 | 0.84 |
| | X | GPR143 | 0.13 | 0.33 | 0.73 |
| Hypo-**methylation** | 11 | IGHMBP2, MRGPRD | 0.81 | 0.78 | 0.14 |

A difference in methylation levels between the normal fetal placentas and maternal blood cells, and a difference in methylation levels between the Down syndrome fetal placentas and normal fetal placentas were compared.

As a result, as shown in Table 5 and FIG. 2, it was confirmed that the MXRA8, MIB2, KIF26B, SP5, ZIC4, ENPEP, PITX2, SH3BP2, SEPP1, FLJ32255, SHROOM1, LINC00574, LOC154449, PRRT4, TMEM176B, MNX1, EGFL7, LOC101928483, NACC2, C9orf69, TLX1, FGF8, TACC2, CPXM2, NKX6-2, TLX1NB, IQSEC3, PCDH8, F7, SOX9, PNMAL2, THBD, MAPK8IP2, KLHDC7B, and GPR143 gene regions were hyper-methylated in the fetal placentas, particularly in Down syndrome fetal placentas. Specifically, the difference in methylation levels of the genes between the normal fetal placentas and maternal blood cells was 10 to 50, and the genes were hyper-methylated in the fetal placentas compared to the maternal blood, and thus, the genes were confirmed to be tissue (placenta)-specific biomarkers. In addition, the difference in methylation levels of the genes between the Down syndrome fetal placentas and normal fetal placentas was 25 to 50, confirming that the genes are disease (Down syndrome)-specific biomarkers hyper-methylated in a Down syndrome fetus compared to a normal fetus. Differences in the methylation levels among the Down syndrome fetal placentas and the other two groups (normal fetal placentas and maternal blood) were all statistically significant (P < 0.05).

In addition, it was confirmed that the IGHMBP2 and MRGPRD gene regions were hypo-methylated in the Down syndrome fetal placentas. It was confirmed that the methylation levels of the IGHMBP2 and MRGPRD gene regions in normal fetal placentas and maternal blood cells were 75 or more, and hyper-methylated, whereas the methylation levels in Down syndrome fetal placentas were 15 or less, and hypo-methylated. Even in this case, differences in the methylation levels among the Down syndrome fetal placentas and the other two groups (normal fetal placentas and maternal blood) were all statistically significant (P < 0.05).

Summarizing the results of Example 4, it may be confirmed that the genes MXRA8, MIB2, KIF26B, SP5, ZIC4, ENPEP, PITX2, SH3BP2, SEPP1, FLJ32255, SHROOM1, LINC00574, LOC154449, PRRT4, TMEM176B, MNX1, EGFL7, LOC101928483, NACC2, C9orf69, TLX1, FGF8, TACC2, CPXM2, NKX6-2, TLX1NB, IQSEC3, PCDH8, F7, SOX9, PNMAL2, THBD, MAPK8IP2, KLHDC7B, and GPR143; and CHODL, NCAM2, CYYR1, GRIK1, OLIG2, CLIC6, SIM2, HLCS, MX2, MX1, TMPRSS2, SLC37A1, PDE9A, CBS, CRYAA, C21orf2, TRPM2, TSPEAR, LINC00162, SSR4P1, SLC19A1, LOC100129027, MCM3AP, YBEY, PRMT2, and ITSN1 in chromosome 21 may be biomarkers that exhibit significant epigenetic characteristics in Down syndrome fetuses compared to the mother and normal fetuses.

Specifically, the DNA methylation levels of the Down syndrome-specific biomarkers present on chromosome 21 are "normal maternal blood: normal fetal placenta: Down syndrome fetal placenta = 0 to 10: 20: 40", and the difference of the methylation levels according to the disease is about 20 %, and when an increase of the numbers of the target chromosome 21 is reflected, it may be confirmed that the final difference in the methylation levels is about 30 %. In addition, the DNA methylation levels of Down syndrome-specific biomarkers present on other chromosomes except for chromosome 21 is "normal maternal blood: normal fetal placenta: Down syndrome fetal placenta = 0 to 10: 30: 70", and it may be confirmed that a difference in the methylation degree according to the disease is about 40 %.

That is, through the above results, it is possible to confirm a specific standard that may be compared with the methylation levels of the mother or normal fetus, which may be used to diagnose a Down syndrome fetus by measuring DNA methylation levels of the biomarker genes.

## Claims

1. A method of providing information for diagnosing Down syndrome, the method comprising:
(a) measuring a methylation level of a Down syndrome biomarker from a biological sample separated from a fetus, wherein the biomarker is at least one first biomarker selected from the group consisting of Table 1 below present on chromosome 21, or a combination with at least one second biomarker selected from the group consisting of Table 2 below present on a chromosome other than chromosome 21,
wherein a methylation level of the biomarker is a methylation level of a base sequence of a region in chromosome of Table 1 below with or without a methylation level of a base sequence of a region in chromosome of Table 2;
**[Table 1]**
| No. | Gene | Region in chromosome |
|---|---|---|
| 1 | CHODL | chr21-19617176-19617247 |
| 2 | | chr21-19617336-19617406 |
| 3 | | chr21-19617673-19617752 |
**[Table 2]**
| No. | Gene | Region in chromosome |
|---|---|---|
| 1 | SHROOM1 | chr5-132158665-132158747 |
| 2 | | chr5-132158828-132158918 |
| 3 | | chr5-132158969-132158984 |
(b) comparing the measured methylation level of the biomarker with a methylation level of the first biomarker, or a combination of the first marker with the second biomarker, of normal cell-free DNA in maternal blood derived from chorionic villus cells; and
(c) determining, by comparing the methylation levels, a presence or a risk of Down syndrome when a difference between the methylation level of a first biomarker of a biological sample separated from a fetus and the methylation level of normal cell-free DNA in maternal blood derived from chorionic villus cells is 10% to 30% or more or additionally when a difference
between the methylation level of a second biomarker of a biological sample separated from a fetus and the methylation level of normal cell-free DNA in maternal blood derived from chorionic villus cells is 30% to 50% or more.

2. The method according to claim 1, wherein the measurement of a methylation level is performed by using any one method selected from the group consisting of PCR, methylation specific PCR, real time methylation specific PCR, PCR using methylated DNA specific binding proteins, quantitative PCR, PCR using methylation specific PNA, melting curve analysis, DNA chip, pyrosequencing, bisulfite sequencing, and methylation next-generation base sequencing.

3. The method according to claim 1, wherein the biological sample separated from a fetus comprises tissues, cells, or cell-free DNA derived from a placenta.

## Patentansprüche

1. Verfahren zur Bereitstellung von Informationen zur Diagnose von Down-Syndrom, wobei das Verfahren umfasst:
(a) Messen eines Methylierungsgrads eines Down-Syndrom Biomarkers aus einer von einem Fötus abgetrennten biologischen Probe, wobei der Biomarker mindestens ein erster Biomarker ist, ausgewählt aus der Gruppe aufgeführt in der nachfolgenden Tabelle 1, die auf Chromosom 21 vorliegt, oder eine Kombination mit mindestens einem zweiten Biomarker, ausgewählt aus der Gruppe aufgeführt in der nachfolgenden Tabelle 2, die auf einem anderen Chromosom als Chromosom 21 vorliegt,
wobei ein Methylierungsgrad des Biomarkers ein Methylierungsgrad einer Basensequenz einer Region in einem Chromosom gemäß nachfolgender Tabelle 1 ist, mit oder ohne einem Methylierungsgrad einer Basensequenz einer Region in einem Chromosom gemäß Tabelle 2;
**[Tabelle 1]**
| Nr. | Gen | Region im Chromosom |
|---|---|---|
| 1 | CHODL | chr21-19617176-19617247 |
| 2 | | chr21-19617336-19617406 |
| 3 | | chr21-19617673-19617752 |
**[Tabelle 2]**
| Nr. | Gen | Region im Chromosom |
|---|---|---|
| 1 | SHROOM1 | chr5-132158665-132158747 |
| 2 | | chr5-132158828-132158918 |
| 3 | | chr5-132158969-132158984 |
(b) Vergleichen des gemessenen Methylierungsgrads des Biomarkers mit einem Methylierungsgrad des ersten Biomarkers oder einer Kombination des ersten Biomarkers mit dem zweiten Biomarker von normaler zellfreier DNA in maternalem Blut, die von Zellen von Chorionzotten stammt; und
(c) Bestimmen, durch Vergleich der Methylierungsgrade, eines Vorliegens oder eines Risikos eines Down-Syndroms, wenn eine Differenz zwischen dem Methylierungsgrad eines ersten Biomarkers einer von einem Fötus abgetrennten biologischen Probe und dem Methylierungsgrad von normaler zellfreier DNA in maternalem Blut, die von Zellen von Chorionzotten stammt, 10% bis 30% oder mehr beträgt, oder zusätzlich, wenn eine Differenz zwischen dem Methylierungsgrad eines zweiten Biomarkers einer von einem Fötus abgetrennten biologischen Probe und dem Methylierungsgrad von normaler zellfreier DNA in maternalem Blut, die von Zellen von Chorionzotten stammt, 30% bis 50% oder mehr beträgt.

2. Verfahren nach Anspruch 1, wobei die Messung eines Methylierungsgrads unter Verwendung eines beliebigen Verfahrens durchgeführt wird, das aus der Gruppe ausgewählt ist, bestehend aus PCR, methylierungsspezifischer PCR, Echtzeitmethylierungsspezifischer PCR, PCR unter Verwendung methylierter DNAspezifischer Bindeproteine, quantitativer PCR, PCR unter Verwendung methylierungsspezifischer PNA, Schmelzkurvenanalyse, DNA-Chip, Pyrosequenzierung, Bisulfit-Sequenzierung und methylierungsspezifischer Sequenzierung der nächsten Generation.

3. Verfahren nach Anspruch 1, wobei die von einem Fötus abgetrennte biologische Probe umfasst Gewebe, Zellen oder zellfreie DNA die von einer Plazenta stammt.

## Revendications

1. Procédé de fourniture d'informations pour diagnostiquer le syndrome de Down, le procédé comprenant:
(a) la mesure d'un niveau de méthylation d'un biomarqueur du syndrome de Down provenant d'un échantillon biologique séparé d'un fœtus, dans lequel le biomarqueur est au moins un premier biomarqueur choisi dans le groupe consistant en le tableau 1 ci-dessous présent sur le chromosome 21, ou d'une combinaison avec au moins un second biomarqueur choisi dans le groupe consistant en le tableau 2 ci-dessous présent sur un chromosome autre que le chromosome 21,
dans lequel un niveau de méthylation du biomarqueur est un niveau de méthylation d'une séquence de bases d'une région dans le chromosome du tableau 1 ci-dessous avec ou sans un niveau de méthylation d'une séquence de bases d'une région dans le chromosome du tableau 2;
**[Tableau 1]**
| N°. | Gène | Région dans le chromosome |
|---|---|---|
| 1 | CHODL | chr21-19617176-19617247 |
| 2 | | chr21-19617336-19617406 |
| 3 | | chr21-19617673-19617752 |
**[Tableau 2]**
| N°. | Gène | Région dans le chromosome |
|---|---|---|
| 1 | SHROOM1 | chr5-132158665-132158747 |
| 2 | | chr5-132158828-132158918 |
| 3 | | chr5-132158969-132158984 |
(b) la comparaison du niveau de méthylation mesuré du biomarqueur avec un niveau de méthylation du premier biomarqueur, ou d'une combinaison du premier marqueur avec le second biomarqueur, d'ADN acellulaire normal dans le sang maternel dérivé de cellules des villosités choriales; et
(c) la détermination, par comparaison des niveaux de méthylation, d'une présence ou d'un risque de syndrome de Down lorsqu'une différence entre le niveau de méthylation d'un premier biomarqueur d'un échantillon biologique séparé d'un fœtus et le niveau de méthylation d'ADN acellulaire normal dans le sang maternel dérivé de cellules des villosités choriales est 10 % à 30 % ou plus, ou encore lorsqu'une différence entre le niveau de méthylation d'un second biomarqueur d'un échantillon biologique séparé d'un fœtus et le niveau de méthylation d'ADN acellulaire normal dans le sang maternel dérivé de cellules des villosités choriales est 30 % à 50 % ou plus.

2. Procédé selon la revendication 1, dans lequel la mesure d'un niveau de méthylation est effectuée à l'aide d'un procédé quelconque choisi dans le groupe consistant en la PCR, la PCR spécifique de la méthylation, la PCR spécifique de la méthylation en temps réel, la PCR utilisant des protéines de liaison spécifiques de l'ADN méthylé, la PCR quantitative, la PCR utilisant un PNA spécifique de la méthylation, l'analyse de courbe de fusion, une puce à ADN, le pyroséquençage, le séquençage au bisulfite et le séquençage de nouvelle génération de bases de méthylation.

3. Procédé selon la revendication 1, dans lequel l'échantillon biologique séparé d'un fœtus comprend des tissus, des cellules ou de l'ADN acellulaire dérivé d'un placenta.
